# EUROPEAN PATENT SPECIFICATION

(11) **EP 4 078 138 B1**
(45) Date of publication and mention of the grant of the patent: **10.07.2024**
(21) Application number: 21824634.6
(22) Date of filing: 30.11.2021
(51) Int. Cl.: G01N 15/02, G01N 15/06, A61M 1/28, A61B 5/145, G01N 15/00

(54) **A CELL COUNTER AND DIAGNOSTIC DEVICE**
ZELLZÄHLER UND DIAGNOSEVORRICHTUNG
COMPTEUR DE CELLULES ET DISPOSITIF DE DIAGNOSTIC

(30) Priority: 30.11.2020 GB 202018831
(43) Date of publication of application: 26.10.2022
(73) Proprietor: Microbiosensor Limited, Manchester M13 9XX (GB)
(72) Inventor: BARKER, Michael Gordon, Manchester Greater Manchester M13 9XX (GB); DOBSON, Curtis, Manchester Greater Manchester M13 9XX (GB); GOVINDJI-BHATT, Nishal, Manchester Greater Manchester M13 9XX (GB); KELL, Darren, Manchester Greater Manchester M13 9XX (GB); HENDERSON, Duncan, Manchester Greater Manchester M13 9XX (GB); KNIGHT, Christopher, Manchester Greater Manchester M13 9XX (GB); GODDARD, Nicholas, Manchester Greater Manchester M13 9XX (GB); SMITH, Martin, Coventry, West Midlands CV8 3HF (GB)
(74) Representative: Appleyard Lees IP LLP
(86) International application number: PCT/GB2021/053122
(87) International publication number: WO 2022/112803

(56) References cited:
- EP-A1- 1 161 670
- EP-A1- 2 869 056
- WO-A1-2019/118929
- JP-A- H09 239 023
- US-A- 4 375 334
- LIU HONGBO ET AL: "Generalized weighted ratio method for accurate turbidity measurement over a wide range", OPTICS EXPRESS, vol. 23, no. 25, 10 December 2015 (2015-12-10), US, pages 32703, XP055893767, ISSN: 1094-4087, DOI: 10.1364/OE.23.032703
- KOCH ET AL: "Some calculations on the turbidity of mitochondria and bacteria", BIOCHIMICA ET BIOPHYSICA ACTA, ELSEVIER, NL, vol. 51, no. 3, 19 August 1961 (1961-08-19), pages 429 - 441, XP024557723, ISSN: 0006-3002, [retrieved on 19610819], DOI: 10.1016/0006-3002(61)90599-6

## Description

The present invention relates to a cell counter and diagnostic device able to be used in a variety of situations, including accompanying treatments such as peritoneal dialysis and laboratory work, where determining the concentration of cells suspended in a medium or physiological liquid or solution is required.

Peritoneal dialysis is a treatment used with human patients where the patient's kidneys are no longer able to perform adequately. The treatment assists in filtering unwanted waste products from the patient's blood. Peritoneal dialysis is used as an alternative to haemodialysis. In some instances, peritoneal dialysis is preferred since it can be performed without medical supervision and in the patient's home. It may also find utility in assisting in the diagnosing of spontaneous bacterial peritonitis in patients with ascites

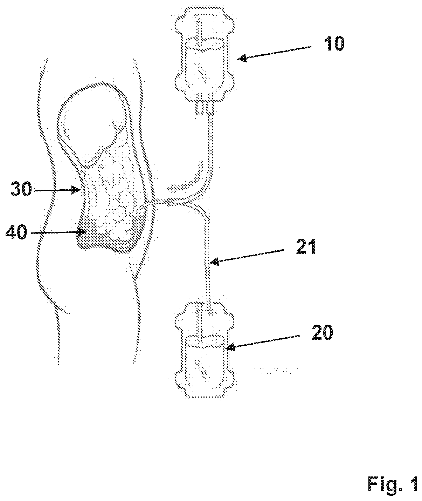

Figure 1 shows a schematic view of a typical peritoneal dialysis set up. The dialysis solution 10 is introduced into the patient's peritoneal cavity 40, via a port, whereby the patient's peritoneal lining 30 acts to filter the patient's blood. The waste effluent fluid 20 is collected for disposal.

A problem with such an arrangement is that it is possible for microbes in the peritoneal cavity to cause peritonitis, which can have life threatening consequences for the patient. It is not currently easy to monitor the levels of microbes (e.g. bacteria or fungi) in the peritoneal cavity and it is an aim of an embodiment of the present invention to address this issue and other issues not outlined here.

In the case of laboratory work, there are various techniques available for determining a cell concentration in a sample. However, many of these are complex and require relatively expensive equipment (e.g. cytometry). It is an aim of an embodiment of the present invention to address shortcomings with such prior art solutions and provide alternative devices.

US 4 375 334 A discloses a device for characterising particle size distribution using the integrated intensity as a function of scattering angle.

According to the present invention there is provided a device as set forth in the appended claims. Other features of the invention will be apparent from the dependent claims, and the description which follows.

In an embodiment, the first and second light intensity readings are taken at displacements proximal the central axis.

In an embodiment, the plurality of displacements are in the range of substantially 1° to 15°, measured from the central axis.

In an embodiment, the detector comprises a plurality of photodetectors, each associated with one of the plurality of displacements.

In an embodiment, the plurality of photodetectors are arranged on both sides of the central axis, such that a first subset of the plurality of displacements is positioned on a first side of the central axis and a second, non-overlapping, subset of the plurality of displacements is positioned on a second side of the central axis.

In an embodiment, the first method uses an Area Under the Curve, AUC, method and the second method uses a Generalised Weighted Ratio, GWR, method.

The sample is contained within a container, wherein the container either contains a static sample or a dynamic, flowing, sample.

In an embodiment, the container is arranged such that light from the light source strikes it an angle sufficient to reduce reflection of light back to the light source.

In an embodiment, the device is arranged to provide user feedback in the form of one or more of a numerical concentration value or a warning that the concentration value exceeds a predefined threshold.

In an embodiment, the user feedback additionally comprises one or more of: an audible signal to the user; a visual signal to the user; and communicating with a remote device to transmit the concentration of cells to the remote device.

According to a second aspect not forming part of the present invention, there is provided a container for use with the device of the first aspect, comprising an entry and an exit, defining respective ends of a flow path, whereby there is provided proximal the entry and the exit a kinked portion arranged to minimise light transmission into the container.

In an embodiment, the cuvette has a male connector at a first end and a female connector at a second end to couple to an effluent line.

According to a third aspect not forming part of the present invention, there is provided a container for use with the device of the first aspect wherein a pair of windows is provided permitting light to enter and exit the container, whereby the pair of windows are recessed from an outer surface such that contact from a user's fingers is prevented or inhibited.

In an embodiment, an exterior of the surface of the container is opaque, except for the pair of windows.

In an embodiment, the container is keyed such that it may only be inserted in one orientation to the device of the first aspect.

Although a few preferred embodiments of the present invention have been shown and described, it will be appreciated by those skilled in the art that various changes and modifications might be made without departing from the scope of the invention, as defined in the appended claims.

For a better understanding of the invention, and to show how embodiments of the same may be carried into effect, reference will now be made, by way of example only, to the accompanying diagrammatic drawings in which:
Figure 1 shows a peritoneal dialysis setup known in the prior art;
Figure 2 shows a schematic representation of an embodiment of the present invention;
Figure 3 shows a representation of light scattering which underlies embodiments of the invention;
Figures 4a-c show details of the arrangement of a cuvette or container, optical source and detectors according to an embodiment of the present invention;
Figure 5a shows a cuvette according to an embodiment of the present invention;
Figure 5b shows another cuvette according to an embodiment of the present invention;
Figure 6 shows a graph illustrating responses according to different concentrations of leukocytes in a sample; and
Figure 7 shows a schematic of certain functional parts of a device according to an embodiment of the invention.

Embodiments of the present invention operate to detect the presence of cells in the effluent fluid 20 during peritoneal dialysis. The cells in question are typically leukocytes. A subset of leukocytes includes neutrophils. Neutrophils above a certain concentration are indicative of the patient fighting an infection. If left unchecked, such an infection can develop into a serious, potentially life threatening, occurrence of peritonitis. Early detection of infection, by the indirect detection of a certain concentration of neutrophils, can be lifesaving. At the very least, it can provide an opportunity for the patient to be treated early, thereby avoiding possible hospital admission and the associated costs and risks of complication. If a bacterial infection is detected, then prompt treatment by a suitable antibiotic can be arranged.

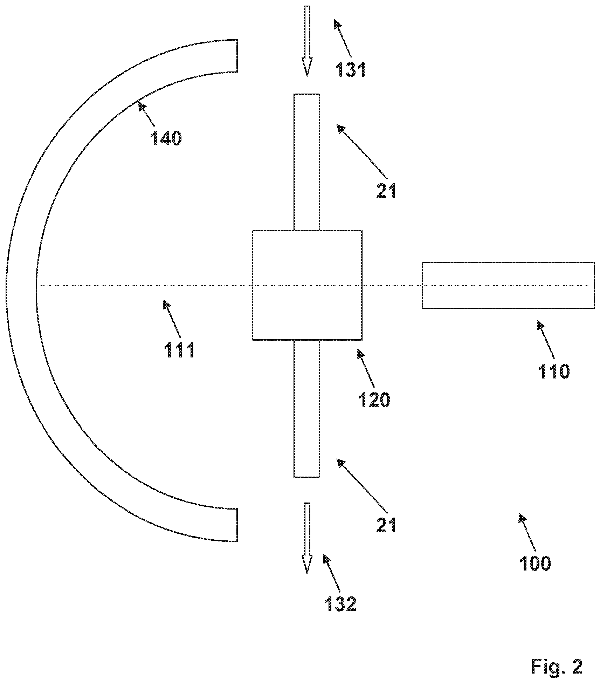

Figure 2 shows a representation of the mode of operation of a first embodiment of the present invention. This is not intended to represent the actual construction of the device and is to illustrate a mode of operation only. The device 100 is arranged to be attached to the effluent waste pipe 21, such that effluent from the peritoneal dialysis process flows through the device 100. Effluent flows in at 131 and flows out at 132 into the effluent reservoir 20.

Interposed between entry port 131 and exit port 132 is a sample-receiving effluent reservoir, container or cuvette 120 which temporarily holds a sample of effluent fluid for analysis. In another embodiment, there is no flow of effluent and a static sample is analysed instead. All other operational details are the same.

In the first (dynamic) embodiment, a flowing sample of effluent (or other fluid) is analysed whilst flowing to the effluent reservoir 20 or other receptacle. In this case, the fluid flows through the analysis device en-route to the reservoir 20 and so the user is not required to dispose of any sample after analysis.

In contrast, in a second (static) embodiment, the sample may be introduced into the cuvette or other suitable receptacle by means of a syringe or similar, whereby the sample is drawn off from or en-route to the effluent reservoir 20. In this case, the sample may be analysed and then disposed of. The second embodiment may be substantially similar to the first embodiment, except that there is no continuous flow of effluent 131, 132. Alternatively, a different form of receptacle may be employed.

One advantage of embodiments of the present invention is that in both the first and second embodiments, the sample for analysis is not required to undergo any preparatory treatment and so embodiments of the invention are well suited for use at home and/or by unskilled persons.

The sample in the cuvette 120, whether static or dynamic, is illuminated via a laser 110. In one embodiment, the laser is a 5mW red laser having a wavelength in the region of 650-700nm. In a preferred embodiment, the LASER is provided in the form of a LASER diode. However, different embodiments may employ different powers and/or wavelengths, as required. In order to achieve the desired effect, a collimating system, such as a lens, may also be required.

Light from the laser or LED 110 passes through the cuvette 120, including the sample, and is detected by a detector 140 located at an opposite side of the cuvette 120. The detector 140 comprises one or more photodiodes which is/are sensitive to light at or about the wavelength transmitted by the laser or LED 110. The detector produces an electrical signal indicative of the strength of the received light. The electrical signal also provides position/angular information indicative of the angular displacement from a central axis 111. In this way, the intensity of light at one or more displacements can be analysed.

The detector is arranged to be exposed to light scattered through the sample. One way to achieve this is to provide a detector in the form of an array of photodiodes, where the number of photodiodes gives a certain detection resolution. An alternative technique employs a CMOS array.

Alternatively, the detector may be arranged to travel along a defined track, measuring light intensity at various points.

The signal recorded at particular locations along the detector 140 gives a measure of the degree of scatter experienced by the light from source 110. The degree of scatter is indicative of the size and concentration of particles in the effluent. In particular, larger particles tend to result in a larger displacement from the axis 111, and the intensity of light at a particular angle is indicative of the number of particles of a particular size. In the case of peritoneal dialysis, the particles of interest are leukocytes having a particular size. The number of such particles determined by measured light intensity at one or more given angular displacements may be compared to a defined threshold to indicate a potential issue for the user in question.

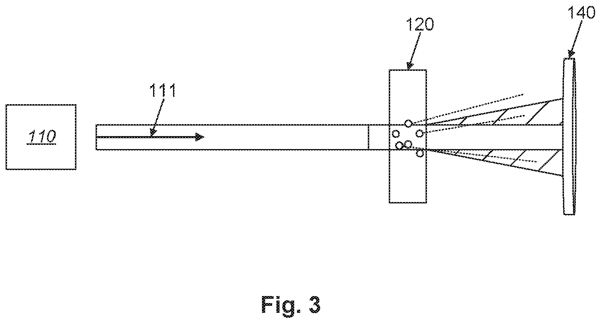

It has been found that the presence of leukocytes in the effluent causes scattering of the laser beam from source 110, away from axis 111. Figure 3 shows a further illustration of the principle involved, whereby light from source 110 illuminates sample cuvette 120, including effluent. In the first embodiment, sample is in the form of a continuous flow and, in the second embodiment, the sample is static. The mode of operation of both embodiments is identical. The light is scattered by the presence of the leukocytes in the sample and causes the light to be scattered, as shown. The degree of scattering is recorded by the detector 140. The degree and intensity of scattering correlates with the concentration of leukocytes in the sample, as described previously.

It is found that in practice, the angle associated with a particular particle size depends on a number of factors including light intensity, the distance between the light source and the detector, path length through the cuvette amongst others. As such, the displacement angle associated with a particular particle size can vary depending upon the precise configuration employed.

It has been found that, in practice, the angular displacement tends to fall in the range of 0 to 15° from the central axis 111. In particular, the range 0 to 12° is found to offer the best range, since readings at displacements above this range tend to be too small to be distinguishable.

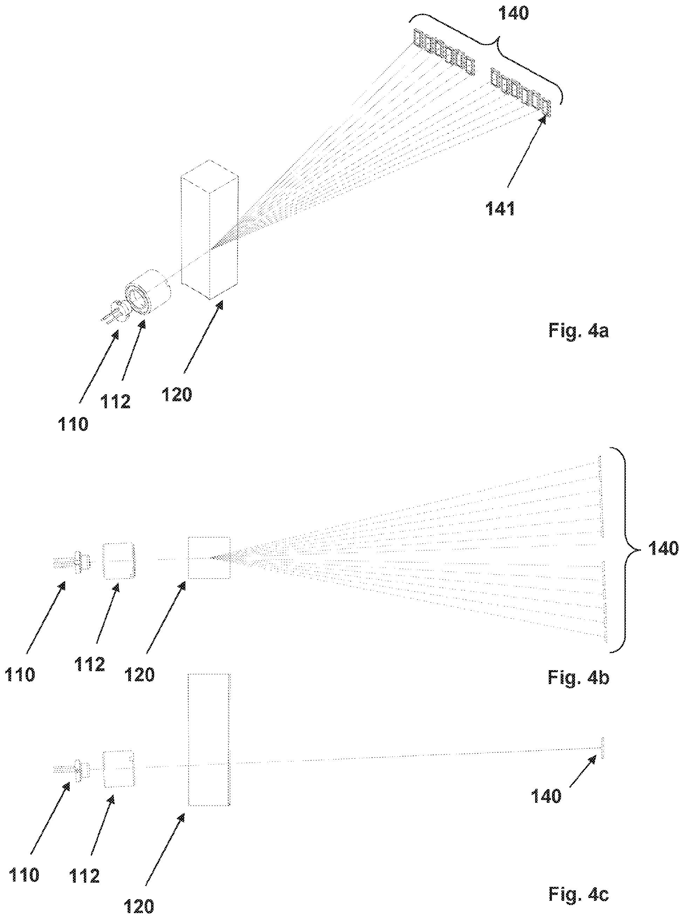

Figures 4a to 4c show details of a configuration of certain parts of the device according to an embodiment of the present invention.

In Figure 4a, the optical source 110 is shown. It emits light towards and through cuvette 120, through a lens 112. The light thereby produced is scattered by the presence of leukocytes in the sample and so the light which leaves the cuvette is scattered somewhat. This scattered light is received at a detector 140. An individual photodetector is a photodiode 141, with a plurality of these forming the overall detector 140. Since each photodetector 141 occupies a finite space, it is difficult to locate many such photodetectors 141 in the space available. Since the scattering caused by the sample is essentially symmetrical about the central axis, on one side of the axis, there is provided a number of photodetectors associated with odd angles of scatter (1°, 3°, 5°, 7°, 9°, 11°) whilst on the other side of the axis, are a number of photodetectors associated with even angles of scatter (2°, 4°, 6°, 8°, 10°, 12°). In this way, more detectors may be provided in a given space, providing greater granularity in the results.

Figures 4b and 4c show, respectively, a top view looking down on the arrangement of Figure 4a and a side view looking at the same arrangement. These figures are provided to illustrate that the light from the source 110 strikes the cuvette at a slight angle, in the region of 2°, which is sufficient to ensure that there is little or no reflection back into the source 120, as this could damage the source and/to adversely affect the results.

A clinical diagnosis of peritonitis is associated with a concentration of 10⁵ cells/ml. Embodiments of the present invention are able to detect a concentration of cells in the region of 10⁴ cells/ml. While such a concentration may be present in a healthy user, the ability to detect changes in the range 10⁴ to 10⁵ cells/ml, offers an opportunity to provide an early warning of infection.

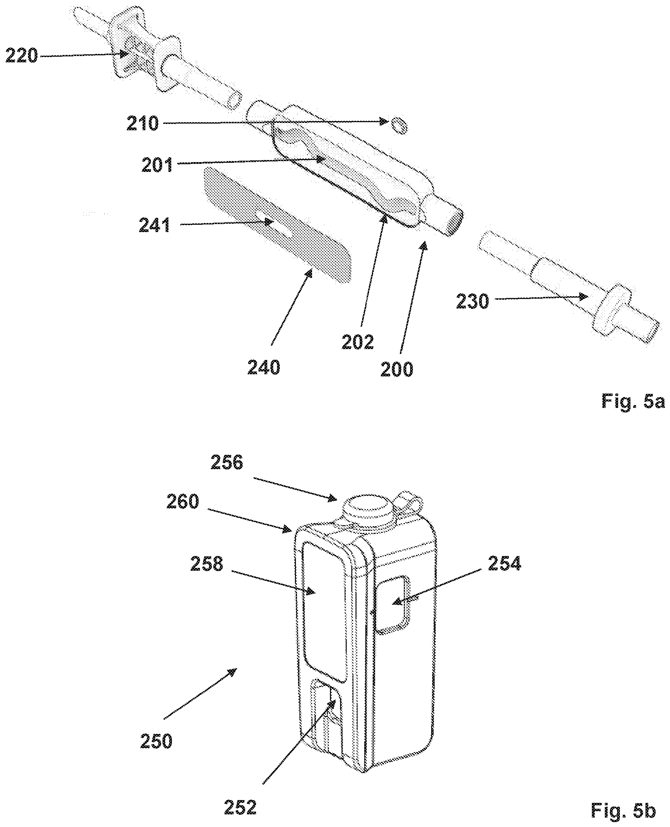

Figure 5a shows a particular form of cuvette 200, which may be employed in place of cuvette 120 shown previously, for use in the first (dynamic) embodiment. The cuvette 200 is connected at one end to a male connector 220 and at a second end to a female connector 230. A flow path 201 is defined within the cuvette in which the effluent or other sample fluid runs. Each of the male and female connectors is attached to the effluent line so that effluent runs through the cuvette 200 and into the reservoir 20.

The cuvette 200 is formed from an opaque plastics material, such that only light from laser or LED 110, which passes through window 210, enters the cuvette. To prevent or minimise unwanted light from entering the cuvette 200 along the flow path, first and second kinks or meanderings 202 are provided proximal each end of the flow path 201 in the cuvette. These have the effect of impeding stray light from the effluent line 21 from entering the cuvette 200 and possibly interfering with the analysis.

The kinks 202 also have the effect of inhibiting the production of bubbles in the flow path which could otherwise interfere with the analysis.

One surface of the cuvette 200 is transparent or less opaque and this is provided with an opaque sticker or seal 240 which is provided with a window or aperture 241 to facilitate the passage of light from laser or LED 110.

The cuvette 200 is intended to be disposable and used once only.

Figure 5b shows a cuvette 250 for use in the static system, where there is no flow of effluent through the cuvette. Here, an effluent sample is introduced into the cuvette via cap 256 located at the top of the cuvette. This ensures that no sample can escape once introduced and the cap is closed. The cuvette 250 takes the form of a cartridge, for insertion into the diagnostic device.

The majority of the cuvette is formed from an opaque plastics material so as to inhibit any stray light from entering the interior of the cuvette and to minimise any internal reflections. However, there are pairs of windows in the opaque plastic, which expose the substantially transparent inner container of the cuvette. The first pair of windows 254 are located towards the top of the cuvette and are provided to permit a simple visual inspection to ensure that sample is present and to an appropriate level. They serve no operational role beyond this.

The second pair of windows 252 are located towards the bottom of the cuvette and are arranged in the central optical axis whereby light from the source 110 passes through the rear and front windows 252 and then towards the detector. In Figure 5b, only one of the pair of windows is visible, but there is a corresponding window on the opposite face of the cuvette 250. This pair of windows 252 are provided in a slightly recessed position in the cuvette. The size of the recess is intended to prevent inadvertent touching of the windows by the user, since grease or other materials on the user's fingers could adversely affect the operation of the device.

The cuvette 250 is provided with a relatively flared front surface 260, whereby the outer diameter of this surface is greater than the outer diameter of the opposing surface. This allows the cuvette to be "keyed" such that it can only be inserted into the diagnostic device in a single orientation. This is since the inner container of the cuvette, which contains the sample does not necessarily have symmetrical optical properties and should be used in a single orientation only, hence the "keyed" arrangement.

Also provided on the front surface of the cuvette is a label 258 which may comprise an identification symbol, such as a barcode or a QR code.

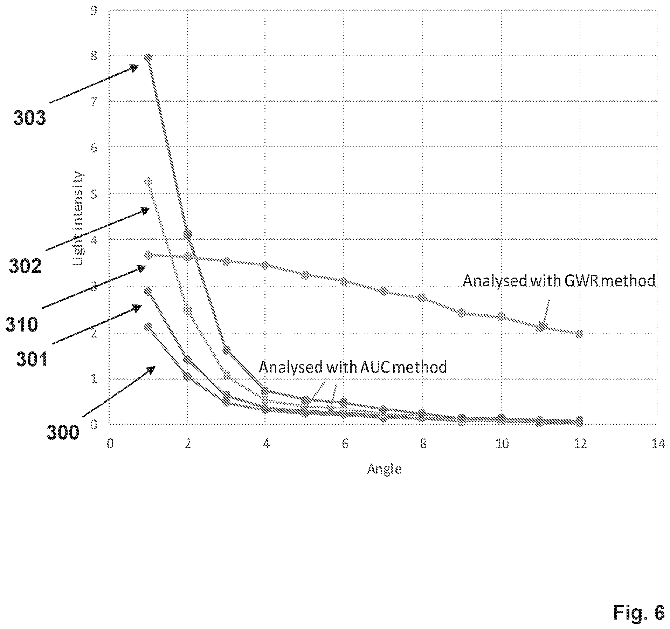

Figure 6 shows a graph illustrating the effect of scattering of the light, from light source 110, through a sample in cases having different concentrations of leukocytes. The x-axis is a measure of the displacement (i.e. the angle of scatter, in degrees) from axis 111. In Figure 6, this runs from 0° to 12°. The y-axis records the intensity of the signal provided by detector 140 and so is a measure of the intensity of light at a particular displacement.

In this particular example, a 5mW Laser is used, having a wavelength in the region 650-700nm. Different wavelengths and powers give different results, and the example given is only one of several options. The skilled person will realise that a different power will lead to different readings of light intensity and a different wavelength will lead to a different response. A wavelength in the range of 650-700nm is found to yield good results in detecting and determining a concentration of leukocytes.

The curves shown represent five different concentrations of leukocytes: 5,000 cells/ml (300); 10,000 cells/ml (301); 25,000 cells/ml (302); 45,000 cells/ml (303); and 10,000,000 cells/ml (310).

As can be seen, the profile of the respective curve follows a regular trajectory from a low concentration (300) to a higher concentration (303). However, when the concentration tends much higher, towards 10⁷ cells/ml (310), the trajectory abruptly changes and the resultant profile ceases to resemble those corresponding to lower concentrations. Essentially, there comes a point where rather than present as a decaying curve (301-303), the curve has a much more linear profile where the gradient is substantially constant across the measured range.

It would be tempting, if one were considering just the first value at the 1° displacement, to think that that value alone is indicative of the concentration of leukocytes. That analysis is true up to a point, but as can be seen by curve 310, this breaks down at some point, when the curve changes form and profile considerably. The first value of curve 310 would appear to indicate a much lower concentration than is actually the case. As such, a more sophisticated approach is required. Such an approach requires more than a single result to be analysed.

In general, the more values of displacement that are analysed, the more accurate the response. At a minimum, two points require analysis. It is convenient to consider the two points closest to the central axis, namely 1° and 2°. It will be noted that the granularity adopted here is 1°, but different embodiments may use a smaller or larger increment and the results will need to be adjusted accordingly. However, the basic principle remains the same and it is instructive to consider the 1° increment set out here.

By examining the first two values of curve 300, it can be seen that the light intensity reading at 1° is a little over 2 and the reading at 2° is approximately 1. As such, it can be seen that the intensity has fallen by about 50% from 1° to 2°. By examining the curves 301 to 303, a similar change in amplitude is observed.

However, by examining the first two values of the curve 310, it can be seen that there is only a slight decrease from the reading at 1° to the reading at 2°. As such, there is clearly a different mechanism at work here and a different approach is required to calculate the associate leukocyte concentration. In this case, the GWR technique is used, as opposed to the AUC technique referred to earlier.

Between the concentration associated with the curve 303 (45,000 cells/ml) and the concentration associated with the curve 310 (10,000,000 cells/ml) the curve profile changes. In order to apply the correct technique, a threshold is determined which dictates which technique of the two is used. It has been found that once the second value (measured at 2°) is less than the first value (measured at 1°) by less than a 25% difference, then the GWR method should be used. If the difference is more than 25%, then the AUC method should be used.

In practice, the absolute value of the threshold may differ, depending upon the particular setup of the apparatus and a threshold value between 10% and 40% may be appropriate with 25% being a preferred value in a particular case only.

In order to account for different practical arrangements, it is instructive to specify the threshold in terms of a percentage decrease per degree of displacement. Hence, in the present example, the threshold may be considered as 25% per degree of displacement. In another embodiment, where intensity readings are taken at intervals of other than 1°, then the calculation may be adjusted accordingly.

Note also, that the best results are achieved proximal to the central axis 111.

An alternative and in, some ways, complementary, technique examines the gradients at two locations on each curve and by means of a comparison therebetween, the correct technique is selected.

Here, an embodiment of the invention is arranged to examine a gradient at one end of the range and compare this with a gradient at another end of the range. In the example shown, the gradient at a first end of the range may be determined by examining the readings from the photodetectors arranged at 1° and 2°, determining a gradient from these two results, and then repeating the process with the readings from the photodetectors arranged at 11° and 12°. In this case, gradient is defined as the difference between two readings, divided the angular displacement between the same readings. The units of intensity are not relevant to this calculation, since a comparison of numerical values is all that is required,

It can be seen that for curves 300-303, the first gradient is relatively steep and so indicative of a relatively lower concentration, whereas the second gradient is relatively flat, which acts to confirm this first approximation. However, for curve 310, associated with a much higher concentration, the first gradient is relatively flat, as is the second one, and so the similarity between them is indicative of this much higher concentration.

Depending on the result of the comparison between the first and second gradient, as set out above, the device is arranged to calculate the concentration of cells using one of two different techniques. If the first gradient is different to the second gradient by more than a defined threshold, then an "area under the curve", AUC, technique is used, whereas if the first and second gradients are substantially the same, within a further defined range, then a "Generalised Weighted Ratio", GWR, technique is used.

Calculating the AUC of the plot of scatter angle vs light intensity can be achieved using any convenient method such as the trapezoidal rule or other methods of integration. Within the appropriate range of cell concentrations, the cell concentration is proportional to the AUC and can be found by suitable calibration with known standards.

The GWR method uses the light intensity recorded at 4 or more scatter angles/displacements and calculates a weighted average intensity. The weighting given to each angle can be found by a number of empirical and computational methods, such as regression analysis, based on measurements of known standards. The weighted average of the light intensities can then be used to calculate the cell concentration in unknown samples within the applicable range of concentrations.

It has been found, empirically, that these two techniques offer better results in different situations, corresponding to different concentrations of leukocytes in the sample under analysis. If the concentration is relatively high, then the GWR method yields better results. However, if the concentration is relatively low, then the AUC method yields better results.

The comparison of gradients referred to above, which determines which method is to be used involves a switching point or threshold. For instance, if the first and second gradients do not differ by more than 25% then this is determined to be substantially the same for the purposes of determining the concentration and so the GWR method is used. However, if the first and second gradients differ by more than the same percentage, then this is determined to be substantially different and so the AUC method is used.

It can be seen from the above that a single reading at relatively small displacements may not be able to adequately distinguish between the different concentrations, and so it is necessary to examine the values recorded at multiple points, as exemplified by studying the first and second gradients.

In an enhanced embodiment, the profile across all photodetectors is compared with one or more pre-stored profiles to better match the observed result with pre-calculated or pre-modelled concentration values. By means of a form of pattern matching in this way, the prior techniques, based on analysing a plurality of points, are implicitly analysed and the result is still affected by these properties of the measured profile.

It is notable that the scattering observed and illustrated in Figure 6 is due to cells of a particular size, which corresponds to the size of leukocytes. Particles of significantly different size will not affect the results. In particular, bacteria, which are significantly smaller, and which may be present in relatively high concentrations in a sample from a patient, do not adversely affect the ability of a device according to an embodiment of the invention to determine a concentration of leukocytes as set out above.

The device 100 may be provided with a display to indicate information to the user regarding the ongoing analysis. In a simple case, this could be in the form of an alert if the cell level is above a predefined threshold. More complex displays may return detailed results, although these may not be suitable or required in all cases.

In a further embodiment, the device 100 may be provided with a form of communication interface whereby the user's results are uploaded to a remote computer system, which may comprise the user's personal records and/or a remote monitoring station whereby problematic results may be reviewed by a physician who may contact the patient in case of concern for theirwellbeing.

The communication interface may comprise a local wireless connection (such as Bluetooth) to a PC, which is then further connected via the internet to the remote computer system. Alternatively, the device 100 may be able to communicate directly with the remote computer system via an integral cellular technology, for instance.

Embodiments of the present invention provide many advantages over prior art solutions. For instance, use of the device requires no specialist knowledge on the part of the user, who is able to use it as an integral part of their dialysis set up.

There is no chemistry involved in the process, meaning that there is no requirement to maintain a stock of indicators, reagents and the like.

The test is performed entirely in situ as part of the process it is monitoring. This means that there is no requirement to send samples to a lab for subsequent analysis.

Any issues which arise during dialysis are flagged instantly to the user and/or a physician (as required).

In the foregoing description, embodiments have been described in the context of peritoneal dialysis. However, other fluids may be analysed. These may include ascitic fluid, urine, sputum, pleural fluid and cerebral spinal fluid.

The foregoing embodiments are focussed on an embodiment of the invention primarily intended for use in a diagnostic setting, possibly in a clinic or a patient's home. However, a further embodiment which operates according to exactly the same principles may be used in a laboratory setting. There is often a need to determine a cell concentration in a sample and the aforementioned embodiment may be used in this way.

In a laboratory setting, the options available to a user may be slightly different to those presented to a patient, since the laboratory worker will typically have a higher skill level in the use of the equipment and may require a different level of detail to a patient. In the aforementioned embodiment, the apparatus is arranged to measure a concentration of leukocytes in particular. However, in a laboratory setting, the user may be interested in analysing other cell types. These may include, for instance, HL60 and Jurkat cells. Measurement of the concentration of these cell types may require an adjustment in the operation of the device, so that different result profiles will require a different processing to that applied to leukocytes. To facilitate this, an additional menu option may be provided so that the user is able to select the most appropriate processing to apply to the particular sample being analysed.

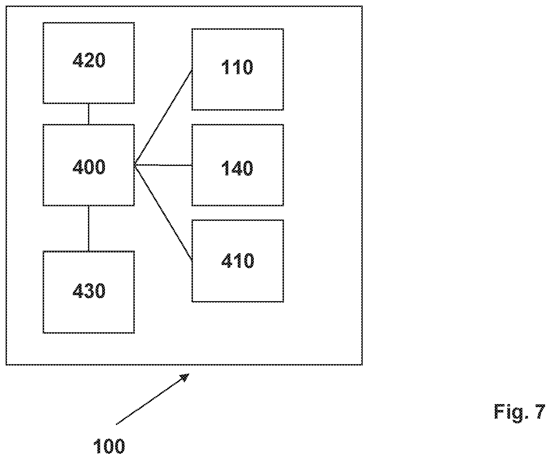

Figure 7 shows, for completeness, a schematic representation of certain functional parts of the device 100 according to an embodiment of the present invention. The device 100 is controlled by processor 400. Processor 400 is any suitable microprocessor or microcontroller, which is programmed to perform the operations required in order to perform the functions set out above. The program which causes the processor 400 to operate is stored in memory 420. The same memory 420 can provide working memory as well as data, such as pre-stored profiles and thresholds used in the analysis of a sample.

The device 100 is operated via power supply 420. This may comprise an internal battery supply or an external mains-operated supply, as required.

The processor 400 is arranged to drive the optical source 110 and to receive signals from the optical detector 140, as set out above.

A result of the analysis is displayed on display 410. The same display may be configured to provide operational information, such as power status, as well as other warning or informational messages for a user.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification.

All of the features disclosed in this specification (including any accompanying claims, abstract and drawings), and/or all of the steps of any method or process so disclosed, may be combined in any combination, except combinations where at least some of such features and/or steps are mutually exclusive.

## Claims

1. A device for determining a concentration of cells of a given size in a sample of fluid, the sample comprising a plurality of cells, comprising:
a light source (110) arranged to emit light along a central axis to illuminate the plurality of cells in the fluid sample;
a container (120, 200, 250) to contain the sample:
a detector (140) arranged to receive light at a plurality of displacements from the central axis the light having passed through the plurality of cells in the fluid sample, and to produce a plurality of signals, each associated with, and indicative of an intensity of the received light at, a respective one of the plurality of displacements; and
a processor (400) arranged to:
obtain a first light intensity reading at a first displacement from the central axis;
obtain a second light intensity reading at a second displacement from the central axis;
determine a difference between the first and second light intensity readings;
if the difference is more than a predefined threshold, determine, using a first method, the concentration of cells of a given size on the basis of the first and second light intensity readings and the difference therebetween and,
if the difference between the first and second light intensity readings is less than the predefined threshold, determine, using a second method, the concentration of cells of the given size on the basis of the first and second light intensity readings and the difference therebetween, wherein the first and second methods are different.

2. The device of claim 1, wherein the device is a diagnostic device.

3. The device of any preceding claim wherein the first and second light intensity readings are taken at displacements proximal the central axis.

4. The device of any preceding claim wherein the detector comprises a plurality of photodetectors, each associated with one of the plurality of displacements.

5. The device of claim 4 wherein the plurality of photodetectors are arranged on both sides of the central axis, such that a first subset of the plurality of displacements is positioned on a first side of the central axis and a second, non-overlapping, subset of the plurality of displacements is positioned on a second side of the central axis.

6. The device of any preceding claim wherein the light source is a laser.

7. The device of claim 6 wherein the laser is a 5 mW red laser having a wavelength in the region of 650-700 nm.

8. The device of any preceding claim wherein the first method uses an Area Under the Curve, AUC, method and the second method uses a Generalised Weighted Ratio, GWR, method.

9. The device of any preceding claim wherein the container either contains a static sample or a dynamic, flowing, sample.

10. The device of claim 9 wherein the container is arranged such that light from the light source strikes it an angle sufficient to reduce reflection of light back to the light source.

11. The device of any preceding claim wherein the device is arranged to provide user feedback in the form of one or more of a numerical concentration value or a warning that the concentration value exceeds a predefined threshold.

12. The device of claim 11 wherein the user feedback additionally comprises one or more of: an audible signal to the user; a visual signal to the user; and communicating with a remote device to transmit the concentration of cells to the remote device.

## Patentansprüche

1. Vorrichtung zum Bestimmen einer Konzentration von Zellen einer bestimmten Größe in einer Fluidprobe, wobei die Probe eine Vielzahl von Zellen umfasst, umfassend:
eine Lichtquelle (110), dazu angeordnet, Licht entlang einer Mittelachse zu emittieren, um die Vielzahl von Zellen in der Fluidprobe zu beleuchten;
einen Behälter (120, 200, 250) zum Enthalten der Probe;
einen Detektor (140), dazu angeordnet, dass er Licht mit einer Vielzahl von Verschiebungen von der Mittelachse aufzunehmen, wobei das Licht durch die Vielzahl von Zellen in der Fluidprobe hindurchgegangen ist, und dazu, eine Vielzahl von Signalen zu erzeugen, die jeweils mit einer entsprechenden der Vielzahl von Verschiebungen verknüpft sind und eine Intensität des empfangenen Lichts bei dieser Verschiebung angeben; und
einen Prozessor (400), angeordnet zum:
Erhalten eines ersten Lichtintensitätsmesswerts bei einer ersten Verschiebung von der Mittelachse;
Erhalten eines zweiten Lichtintensitätsmesswerts bei einer zweiten Verschiebung von der Mittelachse;
Bestimmen einer Differenz zwischen dem ersten und dem zweiten Lichtintensitätsmesswert;
wenn die Differenz größer ist als ein vorgegebener Schwellenwert, Bestimmen, unter Einsatz eines ersten Verfahrens, der Konzentration von Zellen einer bestimmten Größe auf der Grundlage des ersten und des zweiten Lichtintensitätsmesswerts und der Differenz dazwischen, und
wenn die Differenz zwischen dem ersten und dem zweiten Lichtintensitätsmesswert geringer ist als der vorgegebene Schwellenwert, Bestimmen, unter Einsatz eines zweiten Verfahrens, der Konzentration von Zellen der bestimmten Größe auf der Grundlage des ersten und des zweiten Lichtintensitätsmesswert und der Differenz dazwischen, wobei das erste und das zweite Verfahren voneinander verschieden sind.

2. Vorrichtung nach Anspruch 1, wobei die Vorrichtung eine Diagnosevorrichtung ist.

3. Vorrichtung nach einem der vorhergehenden Ansprüche, wobei der erste und der zweite Lichtintensitätsmesswert bei Verschiebungen proximal zu der Mittelachse vorgenommen werden.

4. Vorrichtung nach einem der vorhergehenden Ansprüche, wobei der Detektor eine Vielzahl von Fotodetektoren umfasst, von denen jeder mit einer der Vielzahl von Verschiebungen verknüpft ist.

5. Vorrichtung nach Anspruch 4, wobei die Vielzahl von Fotodetektoren auf beiden Seiten der Mittelachse angeordnet ist, sodass eine erste Untergruppe der Vielzahl von Verschiebungen auf einer ersten Seite der Mittelachse angeordnet ist und eine zweite, nicht überlappende Untergruppe der Vielzahl von Verschiebungen auf einer zweiten Seite der Mittelachse angeordnet ist.

6. Vorrichtung nach einem der vorhergehenden Ansprüche, wobei die Lichtquelle ein Laser ist.

7. Vorrichtung nach Anspruch 6, wobei der Laser ein 5-mW-Rotlaser mit einer Wellenlänge im Bereich von 650 bis 700 nm ist.

8. Vorrichtung nach einem der vorhergehenden Ansprüche, wobei das erste Verfahren ein AUC-Verfahren (Area Under the Curve) und das zweite Verfahren ein GWR-Verfahren (Generalized Weighted Ratio) einsetzt.

9. Vorrichtung nach einem der vorhergehenden Ansprüche, wobei der Behälter entweder eine statische Probe oder eine dynamische, fließende Probe enthält.

10. Vorrichtung nach Anspruch 9, wobei der Behälter so angeordnet ist, dass das Licht von der Lichtquelle in einem ausreichenden Winkel darauf auftrifft, um die Lichtreflexion zurück zu der Lichtquelle zu verringern.

11. Vorrichtung nach einem der vorhergehenden Ansprüche, wobei die Vorrichtung dazu angeordnet ist, dem Benutzer ein Feedback in Form eines oder mehrerer numerischer Konzentrationswerte und/oder eine Warnung darüber bereitzustellen, dass der Konzentrationswert einen vorgegebenen Schwellenwert überschreitet.

12. Vorrichtung nach Anspruch 11, wobei das Benutzerfeedback zusätzlich eines oder mehrere umfasst von: einem akustischen Signal an den Benutzer; einem visuellen Signal an den Benutzer; und Kommunikation mit einer entfernten Vorrichtung, um die Zellkonzentration an die entfernte Vorrichtung zu übertragen.

## Revendications

1. Dispositif destiné à déterminer une concentration de cellules d'une taille donnée dans un échantillon de fluide, l'échantillon comprenant une pluralité de cellules, comprenant :
une source de lumière (110) agencée pour émettre de la lumière le long d'un axe central pour éclairer la pluralité de cellules dans l'échantillon de fluide ;
un récipient (120, 200, 250) pour contenir l'échantillon ;
un détecteur (140) agencé pour recevoir de la lumière à une pluralité de déplacements depuis l'axe central, la lumière ayant traversé la pluralité de cellules dans l'échantillon de fluide, et pour produire une pluralité de signaux, chacun associé à et indicatif d'une intensité de la lumière reçue à un déplacement respectif de la pluralité de déplacements ; et
un processeur (400) agencé pour :
obtenir une première lecture d'intensité lumineuse à un premier déplacement depuis l'axe central ;
obtenir une deuxième lecture d'intensité lumineuse à un deuxième déplacement depuis l'axe central ;
déterminer une différence entre la première et la deuxième lecture d'intensité lumineuse ;
si la différence est supérieure à un seuil prédéfini, déterminer, au moyen d'une première méthode, la concentration de cellules d'une taille donnée sur la base des première et deuxième lectures d'intensité lumineuse et de la différence entre elles et,
si la différence entre la première et la deuxième lecture d'intensité lumineuse est inférieure au seuil prédéterminé, déterminer, au moyen d'une deuxième méthode, la concentration de cellules de la taille donnée sur la base des première et deuxième lectures d'intensité lumineuse et de la différence entre elles, la première et la deuxième méthode étant différentes.

2. Dispositif selon la revendication 1, le dispositif étant un dispositif de diagnostic.

3. Dispositif selon une quelconque revendication précédente dans lequel les première et deuxième lectures d'intensité lumineuse sont prises à des déplacements proches de l'axe central.

4. Dispositif selon une quelconque revendication précédente dans lequel le détecteur comprend une pluralité de photodétecteurs, chacun associé à un déplacement de la pluralité de déplacements.

5. Dispositif selon la revendication 4 dans lequel la pluralité de photodétecteurs sont agencés des deux côtés de l'axe central, de telle sorte qu'un premier sous-ensemble de la pluralité de déplacements est positionné sur un premier côté de l'axe central et un deuxième sous-ensemble, non chevauchant, de la pluralité de déplacements est positionné sur un deuxième côté de l'axe central.

6. Dispositif selon une quelconque revendication précédente dans lequel la source de lumière est un laser.

7. Dispositif selon la revendication 6 dans lequel le laser est un laser rouge de 5 mW ayant une longueur d'onde dans la région de 650-700 nm.

8. Dispositif selon une quelconque revendication précédente, la première méthode utilisant une méthode d'aire sous la courbe, AUC, et la deuxième méthode utilisant une méthode de proportion pondérée généralisée, GWR.

9. Dispositif selon une quelconque revendication précédente dans lequel le récipient contient un échantillon statique ou un échantillon dynamique, en circulation.

10. Dispositif selon la revendication 9 dans lequel le récipient est agencé de telle sorte que la lumière provenant de la source de lumière le frappe à un angle suffisant pour réduire la rétroréflexion de lumière vers la source de lumière.

11. Dispositif selon une quelconque revendication précédente, le dispositif étant agencé pour fournir un retour pour l'utilisateur sous la forme d'une valeur de concentration numérique et/ou d'un avertissement indiquant que la valeur de concentration dépasse un seuil prédéfini.

12. Dispositif selon la revendication 11 dans lequel le retour pour l'utilisateur comprend également un ou plusieurs éléments parmi : un signal sonore pour l'utilisateur ; un signal visuel pour l'utilisateur ; et la communication avec un dispositif distant pour transmettre la concentration de cellules au dispositif distant.
